(19) Europäisches Patentamt
European Patent Office
Office européen des brevets

(11) **EP 4 660 960 A1**

(12) **EUROPEAN PATENT APPLICATION**

(43) Date of publication:
**10.12.2025 Bulletin 2025/50**

(21) Application number: **25173937.1**

(22) Date of filing: **02.05.2025**

(51) International Patent Classification (IPC):
**G06V 10/143** $^{(2022.01)}$     **G06V 10/58** $^{(2022.01)}$
**G06V 10/62** $^{(2022.01)}$     **G06V 10/764** $^{(2022.01)}$
**G06V 20/52** $^{(2022.01)}$

(52) Cooperative Patent Classification (CPC):
**G06V 20/52; G06V 10/143; G06V 10/58;**
**G06V 10/62; G06V 10/764**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **06.06.2024 US 202418736121**

(71) Applicant: **Rebellion Photonics, Inc.**
**Houston, TX 77002 (US)**

(72) Inventors:
• **SHEN, Quan**
  **Charlotte, 28202 (US)**
• **AN, Lingling**
  **Charlotte, 28202 (US)**
• **SHI, Junchuan**
  **Charlotte, 28202 (US)**

(74) Representative: **Houghton, Mark Phillip**
**Patent Outsourcing Limited**
**1 King Street**
**Bakewell, Derbyshire DE45 1DZ (GB)**

(54) **SYSTEMS, APPARATUSES, METHODS, AND COMPUTER PROGRAM PRODUCTS FOR GAS FLARE DISCRIMINATION**

(57)     Systems, apparatuses, methods, and computer program products for gas flare discrimination are provided. An example gas flare discrimination system may comprise at least one gas detection sensor and at least one controller component. In some embodiments, the controller component is configured to obtain image data of a target area. In some embodiments, the controller component is configured to generate, by applying the image data to an acquisition model, gas channel data. In some embodiments, the controller component is configured to generate, by applying the gas channel data to a feature extraction model, gas feature data. In some embodiments, the controller component is configured to generate, by applying the gas feature data to a gas flare discrimination model, a gas flare identification flag. In some embodiments, the controller component is configured to initiate performance of one or more gas flare response actions based at least in part on the gas flare identification flag.

**FIG. 1**

EP 4 660 960 A1

**Description**

**FIELD**

Embodiments of the present disclosure relate generally to systems, apparatuses, methods, and computer program products for gas flare discrimination.

**BACKGROUND**

Applicant has identified many technical challenges and difficulties associated with systems, apparatuses, methods, and computer program products for gas analysis. Through applied effort, ingenuity, and innovation, Applicant has solved problems related to systems, apparatuses, methods, and computer program products for gas analysis by developing solutions embodied in the present disclosure, which are described in detail below.

**BRIEF SUMMARY**

Various embodiments described herein relate to systems, apparatuses, methods, and computer program products for gas flare discrimination.

In accordance with one aspect of the disclosure, a gas flare discrimination is provided. In some embodiments, the gas flare discrimination system may include at least one gas detection sensor. In some embodiments, the gas flare discrimination system may include a controller component. In some embodiments, the controller component is configured to obtain image data of a target area. In some embodiments, the controller component is configured to generate, by applying the image data to an acquisition model, gas channel data. In some embodiments, the controller component is configured to generate, by applying the gas channel data to a feature extraction model, gas feature data. In some embodiments, the controller component is configured to generate, by applying the gas feature data to a gas flare discrimination model, a gas flare identification flag. In some embodiments, the controller component is configured to initiate performance of one or more gas flare response actions based at least in part on the gas flare identification flag.

In some embodiments, the target area is associated with an asset.

In some embodiments, the asset is a processing plant.

In some embodiments, applying the gas feature data to the gas flare discrimination model comprises performing one or more sliding window operations on the gas feature data.

In some embodiments, applying the gas feature data to the gas flare discrimination model comprises performing one or more adaptive threshold operations on the gas feature data.

In some embodiments, the gas flare identification flag is indicative of a gas flare associated with the target area.

In some embodiments, the controller component is configured to generate, by applying the gas feature data to the gas flare discrimination model, a gas leak identification flag.

In some embodiments, the controller component is configured to initiate performance of one or more gas leak response actions based at least in part on the gas flare identification flag.

In some embodiments, the gas leak identification flag is indicative of a gas leak associated with the target area.

In some embodiments, the image data comprises hyperspectral image data of the target area.

In accordance with another aspect of the disclosure, a method is provided. In some embodiments, the method may include obtaining image data of a target area. In some embodiments, the method may include generating, by applying the image data to an acquisition model, gas channel data. In some embodiments, the method may include generating, by applying the gas channel data to a feature extraction model, gas feature data. In some embodiments, the method may include generating, by applying the gas feature data to a gas flare discrimination model, a gas flare identification flag. In some embodiments, the method may include initiating performance of one or more gas flare response actions based at least in part on the gas flare identification flag.

In accordance with another aspect of the disclosure, a computer program product is provided. In some embodiments, the computer program product includes at least one non-transitory computer-readable storage medium having computer program code stored thereon. In some embodiments, the computer program code, in execution with at least one processor, configures the computer program product for obtaining image data of a target area. In some embodiments, the computer program code, in execution with at least one processor, configures the computer program product for generating, by applying the image data to an acquisition model, gas channel data. In some embodiments, the computer program code, in execution with at least one processor, configures the computer program product for generating, by applying the gas channel data to a feature extraction model, gas feature data. In some embodiments, the computer program code, in execution with at least one processor, configures the computer program product for generating, by applying the gas feature data to a gas flare discrimination model, a gas flare identification flag. In some embodiments, the computer program code, in execution with at least one processor, configures the computer program product for initiating performance of one or more gas flare response actions based at least in part on the gas flare identification flag.

## BRIEF DESCRIPTION OF THE DRAWINGS

The description of the illustrative examples may be read in conjunction with the accompanying figures. It will be appreciated that, for simplicity and clarity of illustration, components and elements illustrated in the figures have not necessarily been drawn to scale, unless described otherwise. For example, the dimensions of some of the components or elements may be exaggerated relative to other elements, unless described otherwise. Examples incorporating teachings of the present disclosure are shown and described with respect to the figures presented herein, in which:

FIG. 1 illustrates an example schematic diagram depicting an environment in accordance with various examples of the present disclosure;
FIG. 2 illustrates a schematic diagram depicting an example system in accordance with various examples of the present disclosure;
FIG. 3 illustrates a perspective view of an example gas flare discrimination system in accordance with various examples of the present disclosure;
FIG. 4 illustrates an example controller component of a gas flare discrimination system in accordance with various embodiments of the present disclosure;
FIG. 5 illustrates an example interface in accordance with various embodiments of the present disclosure;
FIG. 6 illustrates a flowchart of an example method in accordance with one or more embodiments of the present disclosure; and
FIG. 7 illustrates a flowchart of another example method in accordance with one or more embodiments of the present disclosure.

## DETAILED DESCRIPTION OF THE INVENTION

Some examples of the present disclosure will now be described more fully hereinafter with reference to the accompanying drawings, in which some, but not all examples of the disclosure are shown. Indeed, these disclosures may be embodied in many different forms and should not be construed as limited to the examples set forth herein; rather, these examples are provided so that this disclosure will satisfy applicable legal requirements. Like numbers refer to like elements throughout.

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may." "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "exemplary" is not necessarily to be construed as preferred or advantageous over other implementations.

The term "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

Overview

Example embodiments disclosed herein address technical problems associated with systems, apparatuses, methods, and computer program products for performing gas analysis. As would be understood by one skilled in the field to which this disclosure pertains, there are numerous example scenarios in which a user may use systems, apparatuses, methods, and computer program products for performing gas analysis.

In many applications, it may be desirable to perform gas analysis. In some examples, it may be desirable to perform gas analysis for an asset, such as a processing plant. In some examples, it may be desirable to perform a gas analysis for multiple target areas (e.g., locations) in an asset. In some examples, it may be desirable to perform a gas analysis to identify gas leaks associated with an asset. In this way, gas leaks can be identified and remedied. Additionally, the amount of gas released into the atmosphere by a gas leak can be quantified to ensure that an asset is adhering to its goals for reducing greenhouse gas emissions.

Example solutions for performing a gas analysis for an asset include identifying gas leaks based on data associated with the asset. In such example solutions, when a gas leak is identified, the asset and/or components of the asset cease operation until the gas leak has been remedied. Additionally, in some example solutions, substantial resources are allocated to remedying the gas leak. As a result, gas leaks decrease the efficiency of an asset (e.g., due to having to cease operations when there is a gas leak) and waste resources (e.g., resources devoted to remedying a gas leak). However, despite the substantial burden caused by gas leaks, such example solutions for performing a gas analysis to identify a gas leak do not contemplate discriminating between gas leaks (e.g., a problem that must be fixed) and gas flares (e.g., a standard operation for many assets). As a result, such example solutions often mistakenly identify gas flares as gas leaks, which is wasteful and inefficient because the asset and/or components of the asset are caused to unnecessarily cease operations and waste resources due to the gas leak. Accordingly, there is a need for systems, apparatuses, methods, and computer program products for gas flare discrimination in order to ensure gas flares are not mistakenly identified as gas leaks.

Thus, to address these and/or other issues related performing a gas analysis, example systems, apparatuses, methods, and computer program products for gas flare discrimination are disclosed herein. For example, an embodiment in this disclosure, described in greater detail below includes a gas flare discrimination system that includes at least one gas detection sensor and a controller component. In some embodiments, the controller component may be configured to obtain image data of a target area. In some embodiments, the controller component may be configured to generate, by applying the image data to an acquisition model, gas channel data. In some embodiments, the controller component may be configured to generate, by applying the gas channel data to a feature extraction model, gas feature data. In some embodiments, the controller component may be configured to generate, by applying the gas feature data to a gas flare discrimination model, a gas flare identification flag. In some embodiments, the controller component may be configured to initiate performance of one or more gas flare response actions based at least in part on the gas flare identification flag. Accordingly, the systems, apparatuses, methods, and computer program products enable gas flare discrimination which enables increases efficiency of an asset and prevents resource waste.

Example Systems and Apparatuses

Embodiments of the present disclosure herein include systems, apparatuses, methods, and computer program products for gas flare discrimination. It should be readily appreciated that the embodiments of the apparatus, systems, methods, and computer program product described herein may be configured in various additional and alternative manners in addition to those expressly described herein.

Gas flare discrimination systems may be utilized in environments in which there is a high risk of gas leaks that may result in fires, explosions and/or acute toxic exposure such as an asset, components of an asset, and/or the like. In this regard, gas flare discrimination systems may be configured to quantify, detect, measure, and/or identify one or more gaseous substances in a particular area or location in order to identify gas leaks and/or gas flares. In some examples, in order to accurately identify gas leaks and/or gas flares, gas flare discrimination systems may be configured to differentiate between gas leaks and gas flares.

One example of a gas flare discrimination system is as a hyperspectral gas flare discrimination system. An example hyperspectral gas flare discrimination system may comprise one or more gas detection sensors (e.g., cameras) that are configured to obtain and analyze raw image/video data (e.g., hyperspectral image data and visible image data) associated with one or more spectral bands (e.g., infrared, visible light) of the electromagnetic spectrum. In some examples, the hyperspectral gas flare discrimination system may be configured to detect one or more gaseous substances including, but not limited to, acetic acid, Ammonia, Benzene, Butadiene, Butane, Ethane, Ethanol, Ethylene, Iso-Butylene, Iso-Pentane, Methane, Methanol, N-Pentane, Propane, Propylene, Toluene, Vinyl Chloride, p- or m-Xylene, and/or the like (e.g., to identify one or more gas leaks) in order to identify gas leaks and/or gas flares.

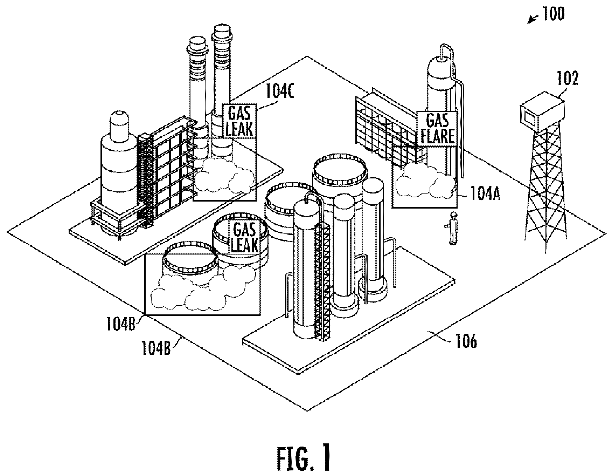

Referring now to FIG. 1, a schematic diagram depicting an environment 100 in accordance with various embodiments of the present disclosure is provided. In some embodiments, the environment 100 may include an asset 106. In some embodiments, for example, the asset 106 may be any type of plant associated with the environment 100. In this regard, the asset 106 may, for example, be a processing plant that receives and processes ingredients as inputs to create a processed product, such as a hydrocarbon processing plant, a refinery, a pulp and paper plant, a chemical plant, an alumina plant, a drilling facility, a fracking field, an oil drilling rig, an offshore platform, a gas sale station, a liquified natural gas vessel, a gas production facility, a gas transmission vehicle, a gas station, and/or the like. Additionally, or alternatively, for example, the asset 106 may include at least one building. In this regard, the asset 106 may, for example, be an industrial building, office building, building associated with a plant, and/or the like.

The asset 106 in some embodiments includes any number of individual components. The components of the asset 106 may perform a particular function during operation of the asset 106. For example, the components may include one or more well components, fracking components, crude processing components, hydrotreating components, isomerization components, vapor recovery components, fluid catalytic cracking components, hydrocracking components, aromatics reduction components, visbreaker components, storage tank components, blender components, pump components, flash venting components, compressor components, cooler components (e.g., air cooler components), sensor components, storage components, flare components, heating, ventilation, and air (HVAC) components, lighting components, and/or the like that perform a particular operation for transforming, storing, releasing, and/or otherwise handling one or more input ingredient(s) (e.g., hydrocarbons, gases, etc.). In this regard, for example, the individual components of a plant may include components associated with a particular process performed by the plant.

In some embodiments, the environment 100 may include a gas flare discrimination system 102. In some embodiments, the gas flare discrimination system 102 may be configured to monitor the environment 100 and/or the asset 106. In some embodiments, the gas flare discrimination system 102 may be positioned within the asset 106 and/or in proximity of the asset 106. In some embodiments, at least a portion of the gas flare discrimination system 102 is moveable with respect to a fixed location such that the gas flare discrimination system 102 can move (e.g., rotate, pan, tilt, and/or the like) to facilitate monitoring of a plurality of target areas within the environment 100 and/or asset 106 (e.g., to identify gas flares and/or gas leaks). As illustrated, the example gas flare discrimination system 102 is configured to monitor at least first target area 104A, a second target area 104B, and a third target area 104C. In this regard, for example, at least a portion of the gas flare discrimination system 102 may be moved (e.g., rotated, paned, titled, and/or the like) to different positions to capture image data. For example, at least a portion of the gas flare discrimination system 102 may be moved (rotated, paned, titled, and/or the like) from a first position associated with the first target area 104A to a second position associated with the second target area 104B (e.g., at least a portion of the gas flare discrimination system 102 may be rotated from the first position to the second position). In some embodiments, a controller component 400 may be configured to cause at least a portion of the gas flare discrimination system 102 (e.g., an at least one gas detection sensor 310) to be moved different positions to capture image data.

In some embodiments, one or more of the plurality of target areas may be associated with a gas flare. For example, the first target area 104A may be associated with a gas flare. Additionally, or alternatively, one or more of the plurality of target areas may be associated with a gas leak. For example, the second target area 104B may be associated with a gas leak.

In various embodiments, the gas flare discrimination system 102 is configured to generate a calibrated image/video stream using a particular light source (e.g., blackbody radiation, infrared radiation, and/or the like) in order to detect an absorption signature of one or more gaseous substances in order to detect one or more gas leaks and/or gas flares. For example, to detect an absorption signature of one or more gaseous substances in order to detect one or more gas leaks and/or one or more gas flares in one or more of the plurality of target areas with the environment 100 and/or asset 106.

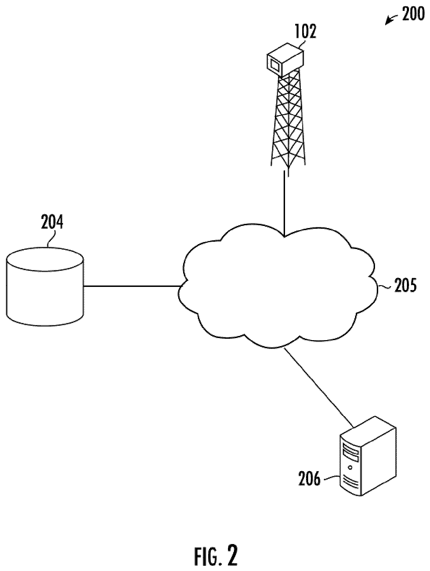

Referring now to FIG. 2, an example schematic diagram depicting an example system 200 in accordance various embodiments of the present disclosure is provided. As depicted, the example system 200 comprises the gas flare discrimination system 102, one or more computing entities 206 (e.g., servers), one or more databases 204, one or more networks 205, and/or the like. In various examples, the system 200 may operate to facilitate monitoring of one or more gaseous substances within a particular location or environment in order to detect one or more gas leaks and/or one or more gas flares. For example, monitoring of one or more gaseous substances indicative of a gas flare and/or a gas leak in a particular location or environment.

In various embodiments, the gas flare discrimination system 102 may be or comprise a hyperspectral gas flare discrimination system that is configured to obtain image data (e.g., video streams) within a location. For example, the gas flare discrimination system 102 may be configured to obtain first image data and/or second image data. In some examples, the gas flare discrimination system 102 may capture image data at a rate of 15 images per second. As discussed above in connection to FIG. 1, the example gas flare discrimination system 102 may be stationary (e.g., mounted on a platform, tower, support structure, and/or the like). In various embodiments, the gas flare discrimination system 102, the one or more databases 204, and/or the one or more user computing entities 108 (e.g., servers) are in electronic communication with each other over the one or more networks 205 such that they can exchange data (e.g., receive and transmit data) with one another (e.g., periodically, and/or in response to requests). Each of the components of the system 200, including the gas flare discrimination system 102, the one or more computing entities 206, and/or the one or more databases 204, may be in communication with one another over the same or different wireless or wired networks 205 including, for example, a wired or wireless Personal Area Network (PAN), Local Area Network (LAN), Metropolitan Area Network (MAN), Wide Area Network (WAN), cellular network, and/or the like. While FIG. 2 illustrates certain system components as separate, standalone devices, the various embodiments are not limited to this particular architecture.

As depicted in FIG. 2, the example system 200 comprises one or more computing entities 206. In general, the terms computing device, entity, device, system, and/or similar words used herein interchangeably may refer to, for example, one or more computers, computing devices, computing entities, desktop computers, mobile phones, tablets, phablets, notebooks, laptops, distributed systems, terminals, servers or server networks, blades, gateways, switches, processing devices, set-top boxes, relays, routers, network access points, base stations, the like, and/or any combination of devices adapted to perform the functions, operations, and/or processes described herein. Such functions, operations, and/or processes may include, for example, transmitting, receiving, operating on, processing, displaying, storing, determining, generating/creating, monitoring, evaluating, comparing, and/or similar terms used herein interchangeably. In one embodiment, these functions, operations, and/or processes can be performed on data, content, information, and/or similar terms used herein interchangeably.

In some examples, the computing entity 206 may also include one or more network and/or communications interfaces for communicating with various computing entities, such as by communicating data, content, information, and/or similar terms used herein interchangeably that can be transmitted, received, operated on, processed, displayed, stored, and/or the like.

In one embodiment, the computing entity 206 may further include or be in communication with non-volatile media (also referred to as non-volatile storage, memory, memory storage, memory circuitry and/or similar terms used herein interchangeably). In one embodiment, the non-volatile storage or memory may include one or more non-volatile storage or memory media as described above, such as hard disks, ROM, PROM, EPROM, EEPROM, flash memory, MMCs, SD memory cards, Memory Sticks, CBRAM, PRAM, FeRAM, RRAM, SONOS, racetrack memory, and/or the like. As will be recognized, the non-volatile storage or memory media may store databases, database instances, database management system entities, data, applications, programs, program modules, scripts, source code, object code, byte code, compiled code, interpreted code, machine code, executable instructions, and/or the like. The term database, database instance, database management system entity, and/or similar terms used herein interchangeably may refer to a structured collection of records or information/data that is stored in a computer-readable storage medium, such as via a relational database, hierarchical database, and/or network database.

In one embodiment, the computing entity 206 may further include or be in communication with volatile media (also referred to as volatile storage, memory, memory storage, memory circuitry and/or similar terms used herein interchangeably). In one embodiment, the volatile storage or memory may also include one or more volatile storage or memory media as described above, such as RAM, DRAM, SRAM, FPM DRAM, EDO DRAM, SDRAM, DDR SDRAM, DDR2 SDRAM, DDR3 SDRAM, RDRAM, RIMM, DIMM, SIMM, VRAM, cache memory, register memory, and/or the like. As will be recognized, the volatile storage or memory media may be used to store at least portions of the databases, database instances, database management system entities, data, applications, programs, program modules, scripts, source code, object code, byte code, compiled code, interpreted code, machine code, executable instructions, and/or the like being executed by, for example, the processing element. Thus, the databases, database instances, database management system entities, data, applications, programs, program modules, scripts, source code, object code, byte code, compiled code, interpreted code, machine code, executable instructions, and/or the like may be used to control certain aspects of the operation of the computing entity 206 with the assistance of the processing element and the operating system.

As indicated, in one embodiment, the computing entity 206 may also include one or more network and/or communications interfaces for communicating with various computing entities, such as by communicating data, content, information, and/or similar terms used herein interchangeably that can be transmitted, received, operated on, processed, displayed, stored, and/or the like. Such communication may be executed using a wired data transmission protocol, such as fiber distributed data interface (FDDI), digital subscriber line (DSL), Ethernet, asynchronous transfer mode (ATM), frame relay, data over cable service interface specification (DOCSIS), or any other wired transmission protocol. Similarly, computing entity 206 may be configured to communicate via wireless external communication networks using any of a variety of protocols, such as general packet radio service (GPRS), Universal Mobile Telecommunications System (UMTS), Code Division Multiple Access 200 (CDMA200), CDMA200 1X (1xRTT), Wideband Code Division Multiple Access (WCDMA), Global System for Mobile Communications (GSM), Enhanced Data rates for GSM Evolution (EDGE), Time Division-Synchronous Code Division Multiple Access (TD-SCDMA), Long Term Evolution (LTE), Evolved Universal Terrestrial Radio Access Network (E-UTRAN), Evolution-Data Optimized (EVDO), High Speed Packet Access (HSPA), High-Speed Downlink Packet Access (HSDPA), IEEE 802.11 (Wi-Fi), Wi-Fi Direct, 802.16 (WiMAX), ultra-wideband (UWB), IR protocols, NFC protocols, RFID protocols, IR protocols, ZigBee protocols, Z-Wave protocols, 6LoWPAN protocols, Wibree, Bluetooth protocols, wireless universal serial bus (USB) protocols, and/or any other wireless protocol. The computing entity 206 may use such protocols and standards to communicate using Border Gateway Protocol (BGP), Dynamic Host Configuration Protocol (DHCP), Domain Name System (DNS), File Transfer Protocol (FTP), Hypertext Transfer Protocol (HTTP), HTTP over TLS/SSL/Secure, Internet Message Access Protocol (IMAP), Network Time Protocol (NTP), Simple Mail Transfer Protocol (SMTP), Telnet, Transport Layer Security (TLS), Secure Sockets Layer (SSL), Internet Protocol (IP), Transmission Control Protocol (TCP), User Datagram Protocol (UDP), Datagram Congestion Control Protocol (DCCP), Stream Control Transmission Protocol (SCTP), HyperText Markup Language (HTML), and/or the like.

As will be appreciated, one or more of the computing entity's 206 components may be located remotely from other computing entity 206 components, such as in a distributed system. Furthermore, one or more of the components may be aggregated and additional components performing functions described herein may be included in the computing entity 206. Thus, the computing entity 206 can be adapted to accommodate a variety of needs and circumstances, such as including various components described with regard to a mobile application executing on a user computing entity, including various input/output interfaces.

As depicted in FIG. 2, any two or more of the illustrative components of the system 200 of FIG. 2 may be configured to communicate with one another via one or more networks 205. The networks 205 may include, but are not limited to, any one or a combination of different types of suitable communications networks such as, for example, cable networks, public networks (e.g., the Internet), private networks (e.g., frame-relay networks), wireless networks, cellular networks, telephone networks (e.g., a public switched telephone network), or any other suitable private and/or public networks. Further, the networks 205 may have any suitable communication range associated therewith and may include, for example, global networks (e.g., the Internet), MANs, WANs, LANs, or PANs. In addition, the networks 205 may include any type of medium over which network traffic may be carried including, but not limited to, coaxial cable, twisted-pair wire, optical fiber, a hybrid fiber coaxial (HFC) medium, microwave terrestrial transceivers, radio frequency communication mediums, satellite communication mediums, or any combination thereof, as well as a variety of network devices and computing platforms provided by network providers or other entities.

While FIG. 2 provides an example system 200, it is noted that the scope of the present disclosure is not limited to the example shown in FIG. 2. In some examples, the system 200 may comprise one or more additional and/or alternative elements, and/or may be different from that illustrated in FIG. 2.

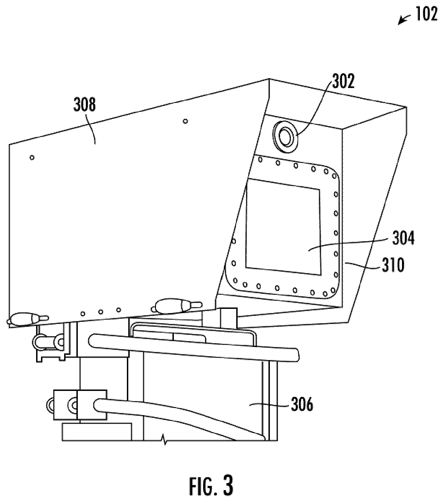

Referring now to FIG. 3, a perspective view of the gas flare discrimination system 102 (e.g., hyperspectral gas flare discrimination system) in accordance various embodiments of the present disclosure is provided. As depicted, the example gas flare discrimination system 102 may comprise a housing 308 configured to contain one or more elements/components of the gas flare discrimination system 102. For example, the housing 308 may include the controller component of the gas flare discrimination system 102. As another example, the housing 308 may include, partially include, and/or partially cover, the at least one gas detection sensor 310 of the gas flare discrimination system 102.

As depicted in FIG. 3, the at least one gas detection sensor 310 of the gas flare discrimination system 102 may include at least one visible gas detection sensor 302 (e.g., an RGB camera). Additionally, or alternatively, the at least one gas detection sensor 310 of the gas flare discrimination system 102 may include at least one hyperspectral gas detection sensor 304. In various embodiments, the gas flare discrimination system 102 is configured to obtain, monitor, and/or capture image data (e.g., infrared image data, visible image data, combinations thereof, and/or the like) via the at least one gas detection sensor 310, such as via the at least one visible gas detection sensor 302 and the at least one hyperspectral gas detection sensor 304. For example, the gas flare discrimination system 102 may be configured to obtain, monitor, and/or capture first image data and/or second image data via the at least one gas detection sensor 310, such as via the at least one visible gas detection sensor 302 and the at least one hyperspectral gas detection sensor 304. In various examples, the gas flare discrimination system 102 is be configured to generate a calibrated image using a particular light source (e.g., blackbody radiation, infrared radiation, and/or the like) in order to detect an absorption signature of one or more gaseous substances in order to detect one or more gas leaks and/or one or more gas flares.

As further depicted in FIG. 3, the gas flare discrimination system 102 comprises a pan-tilt unit 306 that operates to enable movement (e.g., rotations, pans, tilts, and/or the like) of at least a portion of the gas flare discrimination system 102 (e.g., in some examples, 360 degree rotations and/or up to a 45 degree tilt) to facilitate monitoring of more than one target area within a particular location. For example, the housing 308 and/or the at least one gas detection sensor 310 may be moved by the pan-tilt unit 306 to capture first image data from the first target area 104A and second image data from the second target area 104B (e.g., may be moved from a first position associated with the first target area 104A to a second position associated with the second target area 104B). In some embodiments, the example gas flare discrimination system 102 may be mounted on a fixed/stationary support structure (e.g., tower, base, frame, interior building surface, and/or the like) within the environment 100 and/or the asset 106.

While FIG. 3 provides an example gas flare discrimination system 102, it is noted that the scope of the present disclosure is not limited to the example shown in FIG. 3. In some examples, the gas flare discrimination system 102 may comprise one or more additional and/or alternative elements, and/or may be different from that illustrated in FIG. 3. For example, the at least one gas detection sensor 310 may include one or more of an electrochemical sensing component, a catalytic sensing component, and/or a photoionization sensing component.

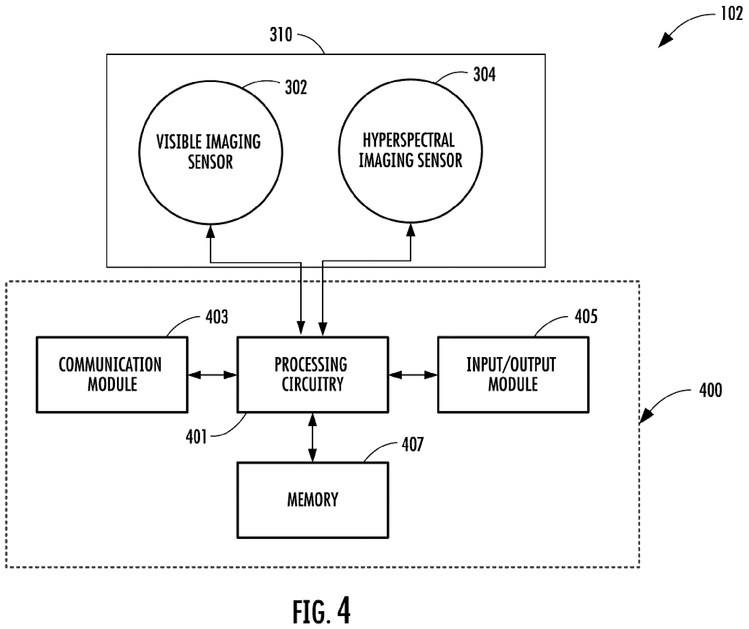

Referring now to FIG. 4, a schematic diagram depicting the controller component 400 of the gas flare discrimination system 102 in electronic communication with various other components in accordance with various embodiments of the present disclosure. As shown, the controller component 400 comprises processing circuitry 401, a communication module 403, input/output module 405, a memory 407, and/or other components configured to perform various operations, procedures, functions or the like described herein.

As shown, the controller component 400 (such as the processing circuitry 401, communication module 403, input/output module 405 and memory 407) is electrically coupled to and/or in electronic communication with at least the at least one gas detection sensor 310, the visible gas detection sensor 302, and/or the hyperspectral gas detection sensor 304. As depicted, the at least one gas detection sensor, the visible gas detection sensor 302, and/or the hyperspectral gas detection sensor 304 may exchange (e.g., transmit and receive) data with the processing circuitry 401 of the controller component 400.

The processing circuitry 401 may be implemented as, for example, various devices comprising one or a plurality of microprocessors with accompanying digital signal processors; one or a plurality of processors without accompanying digital signal processors; one or a plurality of coprocessors; one or a plurality of multi-core processors; one or a plurality of controllers; processing circuits; one or a plurality of computers; and various other processing elements (including integrated circuits, such as ASICs or FPGAs, or a certain combination thereof). In some embodiments, the processing circuitry 401 may comprise one or more processors. In one exemplary embodiment, the processing circuitry 401 is configured to execute instructions stored in the memory 407 or otherwise accessible by the processing circuitry 401. When executed by the processing circuitry 401, these instructions may enable the controller component 400 to execute one or a plurality of the functions as described herein. No matter whether it is configured by hardware, firmware/software methods, or a combination thereof, the processing circuitry 401 may comprise entities capable of executing operations according to the embodiments of the present invention when correspondingly configured. Therefore, for example, when the processing circuitry 401 is implemented as an ASIC, an FPGA, or the like, the processing circuitry 401 may comprise specially configured hardware for implementing one or a plurality of operations described herein. Alternatively, as another example, when the processing circuitry 401 is implemented as an actuator of instructions (such as those that may be stored in the memory 407), the instructions may specifically configure the processing circuitry 401 to execute one or a plurality of algorithms and operations described herein, such as those discussed with reference to FIG. 4.

The memory 407 may comprise, for example, a volatile memory, a non-volatile memory, or a certain combination thereof. Although illustrated as a single memory in FIG. 4, the memory 407 may comprise a plurality of memory components. In various embodiments, the memory 407 may comprise, for example, a hard disk drive, a random access memory, a cache memory, a flash memory, a Compact Disc Read-Only Memory (CD-ROM), a Digital Versatile Disk Read-Only Memory (DVD-ROM), an optical disk, a circuit configured to store information, or a certain combination thereof. The memory 407 may be configured to store information, data, application programs, instructions, and etc., so that the controller component 400 can execute various functions according to the embodiments of the present disclosure. For example, in at least some embodiments, the memory 407 is configured to cache input data for processing by the processing circuitry 401. Additionally, or alternatively, in at least some embodiments, the memory 407 is configured to store program instructions for execution by the processing circuitry 401. The memory 407 may store information in the form of static and/or dynamic information. When the functions are executed, the stored information may be stored and/or used by the controller component 400.

The communication module 403 may be implemented as any apparatus included in a circuit, hardware, a computer program product or a combination thereof, which is configured to receive and/or transmit data from/to another component or apparatus. The computer program product comprises computer-readable program instructions stored on a computer-readable medium (for example, the memory 407) and executed by a controller component 400 (for example, the processing circuitry 401). In some embodiments, the communication module 403 (as with other components discussed herein) may be at least partially implemented as the processing circuitry 401 or otherwise controlled by the processing circuitry 401. In this regard, the communication module 403 may communicate with the processing circuitry 401, for example, through a bus. The communication module 403 may comprise, for example, antennas, transmitters, receivers, transceivers, network interface cards and/or supporting hardware and/or firmware/software, and is used for establishing communication with another apparatus. The communication module 403 may be configured to receive and/or transmit any data that may be stored by the memory 407 by using any protocol that can be used for communication between apparatuses. The communication module 403 may additionally or alternatively communicate with the memory 407, the input/output module 405 and/or any other component of the controller component 400, for example, through a bus.

In some embodiments, the controller component 400 may comprise an input/output module 405. The input/output module 405 may communicate with the processing circuitry 401 to receive instructions input by the user and/or to provide audible, visual, mechanical or other outputs to the user. Therefore, the input/output module 405 may comprise supporting devices, such as a keyboard, a mouse, a display, a touch screen display, and/or other input/output mechanisms. Alternatively, at least some aspects of the input/output module 405 may be implemented on a device used by the user to communicate with the controller component 400. The input/output module 405 may communicate with the memory 407, the communication module 403 and/or any other component, for example, through a bus. One or a plurality of input/output modules and/or other components may be included in the controller component 400.

In some embodiments, the controller component 400 of the gas flare discrimination system 102 may be configured to obtain image data. In some embodiments, image data may be one or more items of data indicative of and/or associated with images of a target area. In this regard, for example, image data may be a data cube indicative of and/or associated with an image of a target area. In some embodiments, image data may include one or more image frames of a target area. In this regard, image data may be a data cube indicative of and/or associated with one or more image frames of a target area.

In some embodiments, image data may include first image data associated with the first target area 104A and/or second image data associated with the second target area 104B. In some embodiments, image data may include hyperspectral image data. In some embodiments, hyperspectral image data may be image data that is associated with a plurality of bands across the electromagnetic spectrum (e.g., infrared, visible light spectrum, x-rays, ultraviolet, combinations thereof, and/or the like). In some embodiments, the hyperspectral image data may be in a time-series format. Additionally, or alternatively, image data may include visible image data. In some embodiments, visible image data may be image data that is associated with the visible light bank of the electromagnetic spectrum.

In some embodiments, the controller component 400 may be configured to obtain image data from the at least one gas detection sensor 310. For example, the controller component 400 may be configured to obtain first image data of the first target area 104A from the at least one gas detection sensor 310. As another example, the controller component 400 may be configured to obtain second image data of the second target area 104B from the at least one gas detection sensor 310. In some embodiments, the controller component 400 may be configured to cause the at least one gas detection sensor 310 to capture image data (e.g., using the visible gas detection sensor 302 and/or the hyperspectral gas detection sensor 304).

In some embodiments, the controller component 400 may be configured to generate gas channel data. In some embodiments, the controller component 400 may be configured to generate gas channel data based at least in part on image data. In this regard, the controller component 400 may be configured to generate gas channel data by applying image data to an acquisition model. In some embodiments, gas channel data may be one or more items of data indicative of and/or associated with one or more properties of image data. In this regard, for example, gas channel data may be one or more items of data indicative of and/or associated with one or more properties of a data cube indicative of and/or associated with images of a target area. For example, gas channel data may be indicative of a range associated with the data cube indicative of and/or associated with images of a target area. As another example, gas channel data may be indicative of a list associated with the data cube indicative of and/or associated with images of a target area. As another example, gas channel data may be indicative of stacked image data from a plurality of cameras.

In some embodiments, the acquisition model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate gas channel data. The acquisition model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques. Said differently, the acquisition model may be configured to use image data to determine one or more items of data indicative of and/or associated with one or more properties of images of a target area. For example, the acquisition model may be configured to use image data of the first target area 104A to determine one or more items of data indicative of and/or associated with one or more properties of images of the first target area 104A.

In some embodiments, the controller component 400 may be configured to generate gas feature data. In some embodiments, the controller component 400 may be configured to generate gas feature data based at least in part on gas channel data. In this regard, the controller component 400 may be configured to generate gas feature data by applying gas channel data to a feature extraction model. In some embodiments, gas feature data may be one or more items of data indicative of and/or associated with one or more determined features of the gas channel data. For example, gas feature data may be indicative of a mean value associated with the gas channel data. As another example, gas feature data may be indicative of a variance associated with the gas channel data. As another example, gas feature data may be indicative of a maximum value associated with the gas channel data. As another example, gas feature data may be indicative of a minimum value associated with the gas channel data. As another example, gas feature data may be indicative of a kernel density estimation associated with the gas channel data. As another example, gas feature data may be indicative of one or more image features associated with the gas channel data. In this regard, for example, gas feature data may be indicative of one or more color features, texture features, shape features, spatial features, frequency domain features, deep features, and/or the like. As another example, gas feature data may be indicative of one or more signal features associated with the gas channel data. In this regard, for example, gas feature data may be indicative of one or more time-domain features, frequency-domain features, time-frequency domain features, statistical features, nonlinear features, energy features, and/or the like.

In some embodiments, the feature extraction model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate gas feature data. The feature extraction model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques. Said differently, the feature extraction model may be configured to use gas channel data to determine one or more items of data indicative of and/or associated with one or more features of gas channel data associated with a target area. For example, the feature extraction model may be configured to use gas channel data associated with the first target area 104A to determine one or more items of data indicative of and/or associated with one or more features of gas channel data associated with the first target area 104A.

In some embodiments, the controller component 400 may be configured to generate a gas flare identification flag. In some embodiments, the controller component 400 may be configured to generate a gas flare identification flag based at least in part on gas feature data and/or gas channel data. In this regard, the controller component 400 may be configured to generate a gas flare identification flag by applying gas feature data and/or gas channel data to a gas flare discrimination model. In some embodiments, a gas flare identification flag may be indicative of a gas flare in the target area. For example, a gas flare identification flag associated with the first target area 104A may indicate that there is a gas flare in the first target area 104A.

In some embodiments, applying gas feature data and/or gas channel data to a gas flare discrimination model may include performing one or more sliding window operations on the gas feature data and/or gas channel data to determine if the gas feature data and/or gas channel data is indicative of a gas flare. In some embodiments, performing a sliding window operation may include performing a sliding window command and/or a sliding window directive on gas feature data and/or gas channel data to determine if gas feature data and/or gas channel data is indicative of a gas flare. For example, a sliding window operation may be performed on gas feature data and/or gas channel data associated with the first target area 104A to determine if the first target area 104A is associated with a gas flare (e.g., a gas flare is present in the first target area 104A). In this regard, for example, when performing a sliding window operation determines that the gas feature data and/or gas channel data is indicative of a gas flare, the controller component 400 may be configured to generate a gas flare identification flag.

Additionally, or alternatively, applying gas feature data and/or gas channel data to a gas flare discrimination model may include performing one or more adaptive threshold operations on the gas feature data and/or gas channel data to determine if the gas feature data and/or gas channel data is indicative of a gas flare. In some embodiments, performing an adaptive threshold operation may include performing an adaptive threshold command and/or an adaptive threshold directive on gas feature data and/or gas channel data to determine if gas feature data and/or gas channel data is indicative of a gas flare. For example, an adaptive threshold operation may be performed on gas feature data and/or gas channel data associated with the second target area 104B to determine if the second target area 104B is associated with a gas flare (e.g., a gas flare is present in the second target area 104B). In this regard, for example, when performing an adaptive threshold operation determines that the gas feature data and/or gas channel data is indicative of a gas flare, the controller component 400 may be configured to generate a gas flare identification flag.

In some embodiments, when the gas channel data is represented by X, the gas feature data in a sliding window w may be represented as equation (1):

$$(1)\ F(X[n - k_1 : n + k_2]) = F(x[n - k_1], x[n - k_1 + 1], \cdots, x[n], \cdots, x[n + k_2 - 1], x[n + k_2])$$

In this regard, in some embodiments, generating a gas flare identification flag by applying gas feature data and/or gas channel data to a gas flare discrimination model may be performed using equation (2):

$$(2)\ Flag_{flare}(n) = \begin{cases} \psi, \ if \ C(F(X[n - k_1 : n + k_2]), \alpha) \\ default, \ otherwise \end{cases}$$

where $x[n]$ is the value of the gas channel data at index $n$, $C(F, \alpha)$ compares the gas feature data F with the adaptive threshold $\alpha$ or a list of adaptive thresholds $\alpha$ (e.g., $\alpha$ may be a single adaptive threshold or a list of adaptive thresholds), $\psi$ is the marker value set when the condition is met, *default* is the default value when the condition is not met, and $w = k_1 + k_2 - 1$. Said differently, in some embodiments, the controller component 400 may be configured to apply gas feature data and/or gas channel data to a gas flare discrimination model to generate a gas flare identification flag ($Flag_{flare}$) by performing one or more sliding window operations and/or one or more adaptive threshold operations using equation (2).

In some embodiments, the controller component 400 may be configured to generate a gas leak identification flag. In some embodiments, the controller component 400 may be configured to generate a gas leak identification flag based at least in part on gas feature data and/or gas channel data. In this regard, the controller component 400 may be configured to generate a gas leak identification flag by applying gas feature data and/or gas channel data to a gas flare discrimination model. In some embodiments, a gas leak identification flag may be indicative of a gas leak in the target area. For example, a gas leak identification flag associated with the first target area 104A may indicate that there is a gas leak in the first target area 104A.

In some embodiments, applying gas feature data and/or gas channel data to a gas flare discrimination model may include performing one or more sliding window operations on the gas feature data and/or gas channel data to determine if the gas feature data and/or gas channel data is indicative of a gas leak. In some embodiments, performing a sliding window operation may include performing a sliding window command and/or a sliding window directive on gas feature data and/or gas channel data to determine if gas feature data and/or gas channel data is indicative of a gas leak. For example, a sliding window operation may be performed on gas feature data and/or gas channel data associated with the first target area 104A to determine if the first target area 104A is associated with a gas leak (e.g., a gas leak is present in the first target area 104A). In this regard, for example, when performing a sliding window operation determines that the gas feature data and/or gas channel data is indicative of a gas leak, the controller component 400 may be configured to generate a gas leak identification flag.

Additionally, or alternatively, applying gas feature data and/or gas channel data to a gas flare discrimination model may include performing one or more adaptive threshold operations on the gas feature data and/or gas channel data to determine if the gas feature data and/or gas channel data is indicative of a gas leak. In some embodiments, performing an adaptive threshold operation may include performing an adaptive threshold command and/or an adaptive threshold directive on gas feature data and/or gas channel data to determine if gas feature data and/or gas channel data is indicative of a gas leak. For example, an adaptive threshold operation may be performed on gas feature data and/or gas channel data associated with the second target area 104B to determine if the second target area 104B is associated with a gas leak (e.g., a gas leak is present in the second target area 104B). In this regard, for example, when performing an adaptive threshold operation determines that the gas feature data and/or gas channel data is indicative of a gas leak, the controller component 400 may be configured to generate a gas leak identification flag.

In some embodiments, when the gas channel data is represented by X, the gas feature data in a sliding window d may be represented as equation (3):

$$(3)\ Q(X[m - i_1 : m + i_2]) = Q(x[m - i_1], x[m - i_1 + 1], \cdots, x[m], \cdots, x[m + i_2 - 1], x[m + i_2])$$

In this regard, in some embodiments, generating a gas leak identification flag by applying gas feature data and/or gas channel data to a gas flare discrimination model may be performed using equation (4):

$$(4)\ Flag_{leak}(m) = \begin{cases} \Theta, & if\ L(Q(X[m - i_1 : m + i_2]), \beta) \\ default, & otherwise \end{cases}$$

where $x[m]$ is the value of the gas channel data at index $m$, $L(Q, \beta)$ compares the gas feature data Q with the adaptive threshold $\beta$ or a list of adaptive thresholds $\beta$ (e.g., $\beta$ may be a single adaptive threshold or a list of adaptive thresholds), $\Theta$ is the marker value set when the condition is met, *default* is the default value when the condition is not met, and d = $i + i_2$ - 1. Said differently, in some embodiments, the controller component 400 may be configured to apply gas feature data and/or gas channel data to a gas flare discrimination model to generate a gas leak identification flag ($Flag_{leak}$) by performing one or more sliding window operations and/or one or more adaptive threshold operations using equation (4).

In some embodiments, the gas flare discrimination model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate a gas flare identification flag and/or a gas leak identification flag. The gas flare discrimination model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques. Said differently, by applying gas feature data to the gas flare discrimination model, the controller component 400 may be configured to determine whether the gas feature data is indicative of a gas flare or a gas leak.

In some embodiments, the controller component 400 may be configured to initiate performance of one or more gas flare response actions. In some embodiments, initiating performance of one or more gas flare response actions may be based at least in part on a gas flare identification flag. In this regard, for example, a gas flare response action may be initiated in response to generation of a gas flare identification flag. In some embodiments, the controller component 400 may be configured to initiate performance of one or more gas flare response actions using a flag handling model. In this regard, the flag handling model may be configured to facilitate performance of one or more gas flare response actions.

In some embodiments, initiating performance of one or more gas flare response actions may include the controller component 400 being configured to trigger an alert that a gas flare identification flag has been generated. In this regard, the controller component 400 may be configured to transmit an alert to one or more computing devices (e.g., one or more remote computing devices) indicating that a gas flare identification flag has been generated.

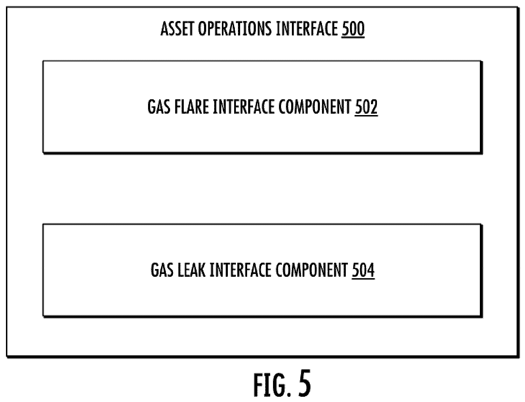

In some embodiments, initiating performance of one or more gas flare response actions may include the controller component 400 being configured to generate a gas flare interface component 502. In some embodiments, the gas flare interface component 502 may include an indication of a gas flare identification flag. Additionally, or alternatively, the gas flare interface component 502 may include an indication of a target area associated with a gas flare identification flag (e.g., third target area 104C). Additionally, or alternatively, the gas flare interface component 502 may include an indication of a component of the asset 106 associated with the gas flare identification flag (e.g., a flaring component of the asset 106). Additionally, or alternatively, the gas flare interface component 502 may include an indication of a time at which a gas flare identification flag was generated. In some embodiments, initiating performance of one or more gas flare response actions may include the controller component 400 being configured to cause the gas flare interface component 502 to be rendered to an asset operations interface 500, such as illustrated in FIG. 5.

In some embodiments, initiating performance of one or more gas flare response actions may include the controller component 400 being configured to cause the asset 106 and/or a component of the asset 106 to cease and/or alter one or more operations being performed by the asset 106 and/or a component of the asset 106. For example, the controller component 400 may be configured to cause a flaring component of the asset 106 to stop flaring gas and/or alter the amount of gas being flared.

In some embodiments, the controller component 400 may be configured to initiate performance of one or more gas leak response actions. In some embodiments, initiating performance of one or more gas leak response actions may be based at least in part on a gas leak identification flag. In this regard, for example, a gas leak response action may be initiated in response to generation of a gas leak identification flag. In some embodiments, the controller component 400 may be configured to initiate performance of one or more gas leak response actions using a flag handling model. In this regard, the flag handling model may be configured to facilitate performance of one or more gas leak response actions.

In some embodiments, initiating performance of one or more gas leak response actions may include the controller component 400 being configured to trigger an alert that a gas leak identification flag has been generated. In this regard, the controller component 400 may be configured to transmit an alert to one or more computing devices (e.g., one or more remote computing devices) indicating that a gas leak identification flag has been generated.

In some embodiments, initiating performance of one or more gas leak response actions may include the controller component 400 being configured to generate a gas leak interface component 504. In some embodiments, the gas leak interface component 504 may include an indication of a gas leak identification flag. Additionally, or alternatively, the gas leak interface component 504 may include an indication of a target area associated with a gas leak identification flag (e.g., third target area 104C). Additionally, or alternatively, the gas leak interface component 504 may include an indication of a component of the asset 106 associated with the gas leak identification flag (e.g., a storage component of the asset 106). Additionally, or alternatively, the gas leak interface component 504 may include an indication of a time at which a gas leak identification flag was generated. In some embodiments, initiating performance of one or more gas leak response actions may include the controller component 400 being configured to cause the gas leak interface component 504 to be rendered to an asset operations interface 500, such as illustrated in FIG. 5.

In some embodiments, initiating performance of one or more gas leak response actions may include the controller component 400 being configured to cause the asset 106 and/or a component of the asset 106 to cease and/or alter one or more operations being performed by the asset 106 and/or a component of the asset 106. For example, the controller component 400 may be configured to cause a pump component of the asset 106 to stop pumping gas and/or alter the amount of gas being pumped.

Example Methods

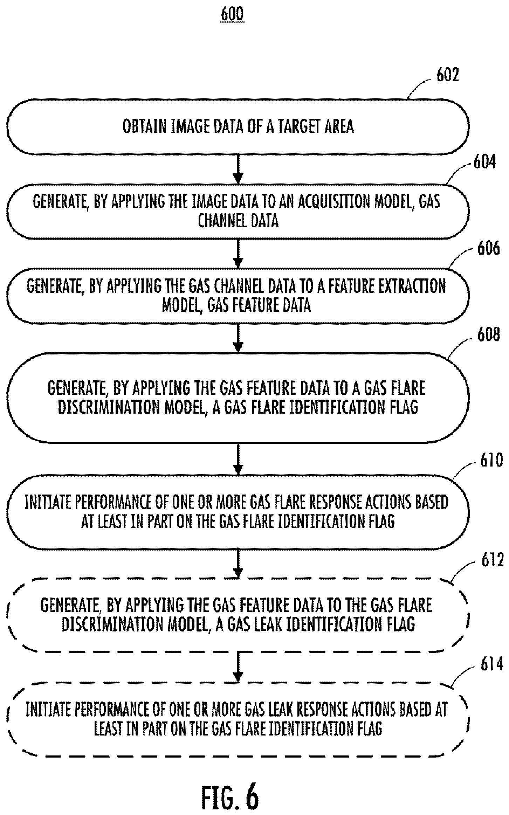

Referring now to FIG. 6, a flowchart diagram illustrating an example method 600 in accordance with various embodiments of the present disclosure is provided. In some embodiments, the method 600 may include one or more operations for gas flare discrimination. In some examples, the method 600 may be performed by a processing circuitry (for example, but not limited to, an application-specific integrated circuit (ASIC), a central processing unit (CPU)). In some examples, the processing circuitry may be electrically coupled to and/or in electronic communication with other circuitries of the example apparatus, such as, but not limited to, a gas flare discrimination system, the controller component of the gas flare discrimination system, a memory (such as, for example, random access memory (RAM) for storing computer program instructions), and/or a display circuitry (for rendering readings on a display).

As shown in block 602, the method 600 may include obtaining image data of a target area. As described above, in some embodiments, image data may be one or more items of data indicative of and/or associated with images of a target area. In this regard, for example, image data may be a data cube indicative of and/or associated with an image of a target area. In some embodiments, image data may include one or more image frames of a target area. In this regard, image data may be a data cube indicative of and/or associated with one or more image frames of a target area.

In some embodiments, image data may include first image data associated with the first target area and/or second image data associated with the second target area. In some embodiments, image data may include hyperspectral image data. In some embodiments, hyperspectral image data may be image data that is associated with a plurality of bands across the electromagnetic spectrum (e.g., infrared, visible light spectrum, x-rays, ultraviolet, combinations thereof, and/or the like). In some embodiments, the hyperspectral image data may be in a time-series format. Additionally, or alternatively, image data may include visible image data. In some embodiments, visible image data may be image data that is associated with the visible light bank of the electromagnetic spectrum.

In some embodiments, the controller component may be configured to obtain image data from the at least one gas detection sensor. For example, the controller component may be configured to obtain first image data of the first target area from the at least one gas detection sensor. As another example, the controller component may be configured to obtain second image data of the second target area from the at least one gas detection sensor. In some embodiments, the controller component may be configured to cause the at least one gas detection sensor to capture image data (e.g., using the visible gas detection sensor and/or the hyperspectral gas detection sensor).

As shown in block 604, the method 600 may include generating, by applying the image data to an acquisition model, gas channel data. As described above, in some embodiments, the controller component may be configured to generate gas channel data based at least in part on image data. In this regard, the controller component may be configured to generate gas channel data by applying image data to an acquisition model. In some embodiments, gas channel data may be one or more items of data indicative of and/or associated with one or more properties of image data. In this regard, for example, gas channel data may be one or more items of data indicative of and/or associated with one or more properties of a data cube indicative of and/or associated with images of a target area. For example, gas channel data may be indicative of a range associated with the data cube indicative of and/or associated with images of a target area. As another example, gas channel data may be indicative of a list associated with the data cube indicative of and/or associated with images of a target area. As another example, gas channel data may be indicative of stacked image data from a plurality of cameras.

In some embodiments, the acquisition model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate gas channel data. The acquisition model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques. Said differently, the acquisition model may be configured to use image data to determine one or more items of data indicative of and/or associated with one or more properties of images of a target area. For example, the acquisition model may be configured to use image data of the first target area to determine one or more items of data indicative of and/or associated with one or more properties of images of the first target area.

As shown in block 606, the method 600 may include generating, by applying the gas channel data to a feature extraction model, gas feature data. As described above, in some embodiments, gas feature data may be one or more items of data indicative of and/or associated with one or more determined features of the gas channel data. For example, gas feature data may be indicative of a mean value associated with the gas channel data. As another example, gas feature data may be indicative of a variance associated with the gas channel data. As another example, gas feature data may be indicative of a maximum value associated with the gas channel data. As another example, gas feature data may be indicative of a minimum value associated with the gas channel data. As another example, gas feature data may be indicative of a kernel density estimation associated with the gas channel data. As another example, gas feature data may be indicative of one or more image features associated with the gas channel data. In this regard, for example, gas feature data may be indicative of one or more color features, texture features, shape features, spatial features, frequency domain features, deep features, and/or the like. As another example, gas feature data may be indicative of one or more signal features associated with the gas channel data. In this regard, for example, gas feature data may be indicative of one or more time-domain features, frequency-domain features, time-frequency domain features, statistical features, nonlinear features, energy features, and/or the like.

In some embodiments, the feature extraction model may be a data entity that describes parameters, hyper-parameters, and/or defined operations of a rules-based, and/or machine learning model (e.g., model including at least one of one or more rule-based layers, one or more layers that depend on trained parameters, coefficients, and/or the like) configured to at least in part generate gas feature data. The feature extraction model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques. Said differently, the feature extraction model may be configured to use gas channel data to determine one or more items of data indicative of and/or associated with one or more features of gas channel data associated with a target area. For example, the feature extraction model may be configured to use gas channel data associated with the first target area to determine one or more items of data indicative of and/or associated with one or more features of gas channel data associated with the first target area.

As shown in block 608, the method 600 may include generating, by applying the gas feature data to a gas flare discrimination model, a gas flare identification flag. As described above, in some embodiments, a gas flare identification flag may be indicative of a gas flare in the target area. For example, a gas flare identification flag associated with the first target area may indicate that there is a gas flare in the first target area.

As shown in block 610, the method 600 may include initiating performance of one or more gas flare response actions based at least in part on the gas flare identification flag. As described above, in some embodiments, a gas flare response action may be initiated in response to generation of a gas flare identification flag. In some embodiments, the controller component may be configured to initiate performance of one or more gas flare response actions using a flag handling model. In this regard, the flag handling model may be configured to facilitate performance of one or more gas flare response actions.

In some embodiments, initiating performance of one or more gas flare response actions may include the controller component being configured to trigger an alert that a gas flare identification flag has been generated. In this regard, the controller component may be configured to transmit an alert to one or more computing devices (e.g., one or more remote computing devices) indicating that a gas flare identification flag has been generated.

In some embodiments, initiating performance of one or more gas flare response actions may include the controller component being configured to generate a gas flare interface component. In some embodiments, the gas flare interface component may include an indication of a gas flare identification flag. Additionally, or alternatively, the gas flare interface component may include an indication of a target area associated with a gas flare identification flag (e.g., third target area). Additionally, or alternatively, the gas flare interface component may include an indication of a component of the asset associated with the gas flare identification flag (e.g., a flaring component of the asset). Additionally, or alternatively, the gas flare interface component may include an indication of a time at which a gas flare identification flag was generated. In some embodiments, initiating performance of one or more gas flare response actions may include the controller component being configured to cause the gas flare interface component to be rendered to an asset operations interface.

In some embodiments, initiating performance of one or more gas flare response actions may include the controller component being configured to cause the asset and/or a component of the asset to cease and/or alter one or more operations being performed by the asset and/or a component of the asset. For example, the controller component may be configured to cause a flaring component of the asset to stop flaring gas and/or alter the amount of gas being flared.

As shown in block 612, the method 600 may optionally include generating, by applying the gas feature data to the gas flare discrimination model, a gas leak identification flag. As described above, in some embodiments, a gas leak identification flag may be indicative of a gas leak in the target area. For example, a gas leak identification flag associated with the first target area may indicate that there is a gas leak in the first target area.

In some embodiments, the gas flare discrimination model may be a data entity that describes parameters, hyperparameters, and/or defined operations of a rules-based, and/or machine learning model (e.g., model including at least one of one or more rule-based layers, one or more layers that depend on trained parameters, coefficients, and/or the like) configured to at least in part generate a gas flare identification flag and/or a gas leak identification flag. The gas flare discrimination model may utilize one or more of any type of machine learning and/or rules-based techniques including one or more of supervised learning (e.g., using user feedback), unsupervised learning, semi-supervised learning, reinforcement learning, computer vision techniques, sequence modeling techniques, language processing techniques, and/or neural network techniques. Said differently, by applying gas feature data to the gas flare discrimination model, the controller component may be configured to determine whether the gas feature data is indicative of a gas flare or a gas leak.

As shown in block 614, the method 600 may optionally include initiating performance of one or more gas leak response actions based at least in part on the gas flare identification flag. As described above, in some embodiments, a gas leak response action may be initiated in response to generation of a gas leak identification flag. In some embodiments, the controller component may be configured to initiate performance of one or more gas leak response actions using a flag handling model. In this regard, the flag handling model may be configured to facilitate performance of one or more gas leak response actions.

In some embodiments, initiating performance of one or more gas leak response actions may include the controller component being configured to trigger an alert that a gas leak identification flag has been generated. In this regard, the controller component may be configured to transmit an alert to one or more computing devices (e.g., one or more remote computing devices) indicating that a gas leak identification flag has been generated.

In some embodiments, initiating performance of one or more gas leak response actions may include the controller component being configured to generate a gas leak interface component. In some embodiments, the gas leak interface component may include an indication of a gas leak identification flag. Additionally, or alternatively, the gas leak interface component may include an indication of a target area associated with a gas leak identification flag (e.g., third target area). Additionally, or alternatively, the gas leak interface component may include an indication of a component of the asset associated with the gas leak identification flag (e.g., a storage component of the asset). Additionally, or alternatively, the gas leak interface component may include an indication of a time at which a gas leak identification flag was generated. In some embodiments, initiating performance of one or more gas leak response actions may include the controller component being configured to cause the gas leak interface component to be rendered to an asset operations interface.

In some embodiments, initiating performance of one or more gas leak response actions may include the controller component being configured to cause the asset and/or a component of the asset to cease and/or alter one or more operations being performed by the asset and/or a component of the asset. For example, the controller component may be configured to cause a pump component of the asset to stop pumping gas and/or alter the amount of gas being pumped.

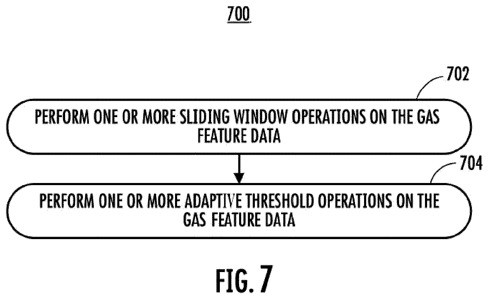

Referring now to FIG. 7, a flowchart diagram illustrating an example method 700 in accordance with various embodiments of the present disclosure is provided. In some embodiments, the method 700 may include one or more operations for gas flare discrimination. For example, the method 700 may include one or more operations for applying gas feature data to a gas flare discrimination model. In some examples, the method 700 may be performed by a processing circuitry (for example, but not limited to, an application-specific integrated circuit (ASIC), a central processing unit (CPU)). In some examples, the processing circuitry may be electrically coupled to and/or in electronic communication with other circuitries of the example apparatus, such as, but not limited to, a gas flare discrimination system, the controller component of the gas flare discrimination system, a memory (such as, for example, random access memory (RAM) for storing computer program instructions), and/or a display circuitry (for rendering readings on a display).

As shown in block 702, the method 700 may include performing one or more sliding window operations on the gas feature data. As shown in block 704, the method 700 may include performing one or more adaptive threshold operations on the gas feature data. As described above, applying gas feature data and/or gas channel data to a gas flare discrimination model may include performing one or more sliding window operations on the gas feature data and/or gas channel data to determine if the gas feature data and/or gas channel data is indicative of a gas flare. In some embodiments, performing a sliding window operation may include performing a sliding window command and/or a sliding window directive on gas feature data and/or gas channel data to determine if gas feature data and/or gas channel data is indicative of a gas flare. For example, a sliding window operation may be performed on gas feature data and/or gas channel data associated with the first target area to determine if the first target area is associated with a gas flare (e.g., a gas flare is present in the first target area). In this regard, for example, when performing a sliding window operation determines that the gas feature data and/or gas channel data is indicative of a gas flare, the controller component may be configured to generate a gas flare identification flag.

Additionally, or alternatively, applying gas feature data and/or gas channel data to a gas flare discrimination model may include performing one or more adaptive threshold operations on the gas feature data and/or gas channel data to determine if the gas feature data and/or gas channel data is indicative of a gas flare. In some embodiments, performing an adaptive threshold operation may include performing an adaptive threshold command and/or an adaptive threshold directive on gas feature data and/or gas channel data to determine if gas feature data and/or gas channel data is indicative of a gas flare. For example, an adaptive threshold operation may be performed on gas feature data and/or gas channel data associated with the second target area to determine if the second target area is associated with a gas flare (e.g., a gas flare is present in the second target area). In this regard, for example, when performing an adaptive threshold operation determines that the gas feature data and/or gas channel data is indicative of a gas flare, the controller component may be configured to generate a gas flare identification flag.

In some embodiments, when the gas channel data is represented by X, the gas feature data in a sliding window w may be represented as equation (5):

$$(5)\ F(X[n - k_1 : n + k_2]) = F(x[n - k_1], x[n - k_1 + 1], \cdots, x[n], \cdots, x[n + k_2 - 1], x[n + k_2])$$

In this regard, in some embodiments, generating a gas flare identification flag by applying gas feature data and/or gas channel data to a gas flare discrimination model may be performed using equation (6):

$$(6)\ Flag_{flare}(n) = \begin{cases} \psi, \ if \ C(F(X[n - k_1 : n + k_2]), \alpha) \\ default, \ otherwise \end{cases}$$

where $x[n]$ is the value of the gas channel data at index $n$, $C(F, \alpha)$ compares the gas feature data $F$ with the adaptive threshold $\alpha$ or a list of adaptive thresholds $\alpha$ (e.g., $\alpha$ may be a single adaptive threshold or a list of adaptive thresholds), $\psi$ is the marker value set when the condition is met, *default* is the default value when the condition is not met, and $w = k_1 + k_2 - 1$. Said differently, in some embodiments, the controller component 400 may be configured to apply gas feature data and/or gas channel data to a gas flare discrimination model to generate a gas flare identification flag ($Flag_{flare}$) by performing one or more sliding window operations and/or one or more adaptive threshold operations using equation (6).

In some embodiments, the controller component may be configured to generate a gas leak identification flag. In some embodiments, the controller component may be configured to generate a gas leak identification flag based at least in part on gas feature data and/or gas channel data. In this regard, the controller component may be configured to generate a gas leak identification flag by applying gas feature data and/or gas channel data to a gas flare discrimination model. In some embodiments, a gas leak identification flag may be indicative of a gas leak in the target area. For example, a gas leak identification flag associated with the first target area may indicate that there is a gas leak in the first target area.

In some embodiments, applying gas feature data and/or gas channel data to a gas flare discrimination model may include performing one or more sliding window operations on the gas feature data and/or gas channel data to determine if the gas feature data and/or gas channel data is indicative of a gas leak. In some embodiments, performing a sliding window operation may include performing a sliding window command and/or a sliding window directive on gas feature data and/or gas channel data to determine if gas feature data and/or gas channel data is indicative of a gas leak. For example, a sliding window operation may be performed on gas feature data and/or gas channel data associated with the first target area to determine if the first target area is associated with a gas leak (e.g., a gas leak is present in the first target area). In this regard, for example, when performing a sliding window operation determines that the gas feature data and/or gas channel data is indicative of a gas leak, the controller component may be configured to generate a gas leak identification flag.

Additionally, or alternatively, applying gas feature data and/or gas channel data to a gas flare discrimination model may include performing one or more adaptive threshold operations on the gas feature data and/or gas channel data to determine if the gas feature data and/or gas channel data is indicative of a gas leak. In some embodiments, performing an adaptive threshold operation may include performing an adaptive threshold command and/or an adaptive threshold directive on gas feature data and/or gas channel data to determine if gas feature data and/or gas channel data is indicative of a gas leak. For example, an adaptive threshold operation may be performed on gas feature data and/or gas channel data associated with the second target area to determine if the second target area is associated with a gas leak (e.g., a gas leak is present in the second target area). In this regard, for example, when performing an adaptive threshold operation determines that the gas feature data and/or gas channel data is indicative of a gas leak, the controller component may be configured to generate a gas leak identification flag.

In some embodiments, when the gas channel data is represented by X, the gas feature data in a sliding window d may be represented as equation (7):

$$(7)\ Q(X[m - i_1 : m + i_2]) = Q(x[m - i_1], x[m - i_1 + 1], \cdots, x[m], \cdots, x[m + i_2 - 1], x[m + i_2])$$

In this regard, in some embodiments, generating a gas leak identification flag by applying gas feature data and/or gas channel data to a gas flare discrimination model may be performed using equation (8):

$$(8)\ Flag_{leak}(m) = \begin{cases} \Theta, & if\ L(Q(X[m - i_1 : m + i_2]), \beta) \\ default, & otherwise \end{cases}$$

where $x[m]$ is the value of the gas channel data at index $m$, $L(Q, \beta)$ compares the gas feature data $Q$ with the adaptive threshold $\beta$ or a list of adaptive thresholds $\beta$ (e.g., $\beta$ may be a single adaptive threshold or a list of adaptive thresholds), $\Theta$ is the marker value set when the condition is met, *default* is the default value when the condition is not met, and $d = i + i_2 - 1$. Said differently, in some embodiments, the controller component 400 may be configured to apply gas feature data and/or gas channel data to a gas flare discrimination model to generate a gas leak identification flag ($Flag_{leak}$) by performing one or more sliding window operations and/or one or more adaptive threshold operations using equation (8).

Operations and/or functions of the present disclosure have been described herein, such as in flowcharts. As will be appreciated, computer program instructions may be loaded onto a computer or other programmable apparatus (e.g., hardware) to produce a machine, such that the resulting computer or other programmable apparatus implements the operations and/or functions described in the flowchart blocks herein. These computer program instructions may also be stored in a computer-readable memory that may direct a computer, processor, or other programmable apparatus to operate and/or function in a particular manner, such that the instructions stored in the computer-readable memory produce an article of manufacture, the execution of which implements the operations and/or functions described in the flowchart blocks. The computer program instructions may also be loaded onto a computer, processor, or other programmable apparatus to cause a series of operations to be performed on the computer, processor, or other programmable apparatus to produce a computer-implemented process such that the instructions executed on the computer, processor, or other programmable apparatus provide operations for implementing the functions and/or operations specified in the flowchart blocks. The flowchart blocks support combinations of means for performing the specified operations and/or functions and combinations of operations and/or functions for performing the specified operations and/or functions. It will be understood that one or more blocks of the flowcharts, and combinations of blocks in the flowcharts, can be implemented by special purpose hardware-based computer systems which perform the specified operations and/or functions, or combinations of special purpose hardware with computer instructions.

While this specification contains many specific embodiments and implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

While operations and/or functions are illustrated in the drawings in a particular order, this should not be understood as requiring that such operations and/or functions be performed in the particular order shown or in sequential order, or that all illustrated operations be performed, to achieve desirable results. In certain circumstances, operations and/or functions in alternative ordering may be advantageous. In some cases, the actions recited in the claims may be performed in a different order and still achieve desirable results. Thus, while particular embodiments of the subject matter have been described, other embodiments are within the scope of the following claims.

While this specification contains many specific embodiment and implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are illustrated in the drawings in a particular order, this should not be understood as requiring that such operations be performed in the particular order shown or in sequential order, or that all illustrated operations be performed, to achieve desirable results. In certain circumstances, operations in alternative ordering may be advantageous. In some cases, the actions recited in the claims may be performed in a different order and still achieve desirable results.

**Claims**

1. A gas flare discrimination system comprising:

   at least one gas detection sensor; and
   a controller component, wherein the controller component is configured to:

      obtain image data of a target area;
      generate, by applying the image data to an acquisition model, gas channel data;
      generate, by applying the gas channel data to a feature extraction model, gas feature data;
      generate, by applying the gas feature data to a gas flare discrimination model, a gas flare identification flag; and
      initiate performance of one or more gas flare response actions based at least in part on the gas flare identification flag.

2. The gas flare discrimination system of claim 1, wherein the target area is associated with an asset.

3. The gas flare discrimination system of claim 2, wherein the asset is a processing plant.

4. The gas flare discrimination system of claim 1, wherein applying the gas feature data to the gas flare discrimination model comprises performing one or more sliding window operations on the gas feature data.

5. The gas flare discrimination system of claim 1, wherein applying the gas feature data to the gas flare discrimination model comprises performing one or more adaptive threshold operations on the gas feature data.

6. The gas flare discrimination system of claim 1, wherein the gas flare identification flag is indicative of a gas flare associated with the target area.

**7.** The gas flare discrimination system of claim 1, wherein the controller component is further configured to:
generate, by applying the gas feature data to the gas flare discrimination model, a gas leak identification flag.

**8.** The gas flare discrimination system of claim 7, wherein the controller component is further configured to:
initiate performance of one or more gas leak response actions based at least in part on the gas flare identification flag.

**9.** The gas flare discrimination system of claim 7, wherein the gas leak identification flag is indicative of a gas leak associated with the target area.

**10.** The gas flare discrimination system of claim 1, wherein the image data comprises hyperspectral image data of the target area.

**11.** A method comprising:

obtaining image data of a target area;
generating, by applying the image data to an acquisition model, gas channel data;
generating, by applying the gas channel data to a feature extraction model, gas feature data;
generating, by applying the gas feature data to a gas flare discrimination model, a gas flare identification flag; and
initiating performance of one or more gas flare response actions based at least in part on the gas flare identification flag.

**12.** The method of claim 11, wherein the target area is associated with an asset, wherein the asset is a processing plant.

**13.** The method of claim 11, wherein applying the gas feature data to the gas flare discrimination model comprises performing one or more sliding window operations on the gas feature data.

**14.** The method of claim 11, wherein applying the gas feature data to the gas flare discrimination model comprises performing one or more adaptive threshold operations on the gas feature data.

**15.** A computer program product comprising at least one non-transitory computer-readable storage medium having computer program code stored thereon that, in execution with at least one processor, configures the computer program product for:

obtaining image data of a target area;
generating, by applying the image data to an acquisition model, gas channel data;
generating, by applying the gas channel data to a feature extraction model, gas feature data;
generating, by applying the gas feature data to a gas flare discrimination model, a gas flare identification flag; and
initiating performance of one or more gas flare response actions based at least in part on the gas flare identification flag.

FIG. 1

FIG. 2

FIG. 3

FIG. 4

EP 4 660 960 A1

ASSET OPERATIONS INTERFACE 500

GAS FLARE INTERFACE COMPONENT 502

GAS LEAK INTERFACE COMPONENT 504

FIG. 5

600

OBTAIN IMAGE DATA OF A TARGET AREA ⟶ 602

GENERATE, BY APPLYING THE IMAGE DATA TO AN ACQUISITION MODEL, GAS CHANNEL DATA ⟶ 604

GENERATE, BY APPLYING THE GAS CHANNEL DATA TO A FEATURE EXTRACTION MODEL, GAS FEATURE DATA ⟶ 606

GENERATE, BY APPLYING THE GAS FEATURE DATA TO A GAS FLARE DISCRIMINATION MODEL, A GAS FLARE IDENTIFICATION FLAG ⟶ 608

INITIATE PERFORMANCE OF ONE OR MORE GAS FLARE RESPONSE ACTIONS BASED AT LEAST IN PART ON THE GAS FLARE IDENTIFICATION FLAG ⟶ 610

GENERATE, BY APPLYING THE GAS FEATURE DATA TO THE GAS FLARE DISCRIMINATION MODEL, A GAS LEAK IDENTIFICATION FLAG ⟶ 612

INITIATE PERFORMANCE OF ONE OR MORE GAS LEAK RESPONSE ACTIONS BASED AT LEAST IN PART ON THE GAS FLARE IDENTIFICATION FLAG ⟶ 614

FIG. 6

700

702

PERFORM ONE OR MORE SLIDING WINDOW OPERATIONS ON THE GAS FEATURE DATA

704

PERFORM ONE OR MORE ADAPTIVE THRESHOLD OPERATIONS ON THE GAS FEATURE DATA

FIG. 7

Europäisches
Patentamt

European
Patent Office

Office européen
des brevets

# EUROPEAN SEARCH REPORT

**Application Number**

EP 25 17 3937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/046172 A1 (RAO VENKATAGIRI S [IN] ET AL) 19 February 2009 (2009-02-19) | 1-4,6, 11-13,15 | INV. G06V10/143 |
| Y | * paragraph [0002] - paragraph [0023] * | 5,7-10, 14 | G06V10/58 G06V10/62 G06V10/764 |
| Y | ----- WO 2020/247664 A1 (HONEYWELL INT INC [US]) 10 December 2020 (2020-12-10) * paragraph [0009] - paragraph [0021] * | 5,14 | G06V20/52 |
| Y | ----- AU 2021 336 432 A1 (CAMERON TECH LTD [NL]) 6 April 2023 (2023-04-06) * paragraph [0028] - paragraph [0045] * | 7-9 | |
| Y | ----- US 2013/342680 A1 (ZENG YOUSHENG [US] ET AL) 26 December 2013 (2013-12-26) * paragraphs [0018] - [0020], [0036] * ----- | 10 | |

**TECHNICAL FIELDS SEARCHED (IPC)**

G06V

The present search report has been drawn up for all claims

| Place of search | Date of completion of the search | Examiner |
|---|---|---|
| The Hague | 17 September 2025 | Natanni, Francesco |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...........................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 17 3937

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2025

| Patent document cited in search report | Publication date | Patent family member(s) | | Publication date |
|---|---|---|---|---|
| US 2009046172 A1 | 19-02-2009 | NONE | | |
| WO 2020247664 A1 | 10-12-2020 | US | 2020386404 A1 | 10-12-2020 |
| | | WO | 2020247664 A1 | 10-12-2020 |
| AU 2021336432 A1 | 06-04-2023 | AU | 2021336432 A1 | 06-04-2023 |
| | | CA | 3194023 A1 | 10-03-2022 |
| | | CN | 116324862 A | 23-06-2023 |
| | | GB | 2613296 A | 31-05-2023 |
| | | NO | 20230213 A1 | 02-03-2023 |
| | | SA | 523442804 B1 | 13-11-2024 |
| | | US | 2022065834 A1 | 03-03-2022 |
| | | WO | 2022051572 A1 | 10-03-2022 |
| US 2013342680 A1 | 26-12-2013 | NONE | | |

EPO FORM P0459

For more details about this annex : see Official Journal of the European Patent Office, No. 12/82